(19) Europäisches Patentamt
European Patent Office
Office européen des brevets

(11) Veröffentlichungsnummer: **0 168 766**
**B1**

(12)
# EUROPÄISCHE PATENTSCHRIFT

(45) Veröffentlichungstag der Patentschrift: **16.08.89**

(51) Int. Cl.⁴: **C 07 D 253/06**, C 07 D 251/38, C 07 D 239/56, C 07 C 157/14

(21) Anmeldenummer: **85108577.9**

(22) Anmeldetag: **10.07.85**

(54) **Verfahren zur Herstellung S-substituierter Isothioharnstoffe.**

(30) Priorität: **18.07.84 US 632132**

(43) Veröffentlichungstag der Anmeldung: **22.01.86 Patentblatt 86/4**

(45) Bekanntmachung des Hinweises auf die Patenterteilung: **16.08.89 Patentblatt 89/33**

(84) Benannte Vertragsstaaten: **AT BE CH DE FR GB IT LI NL**

(56) Entgegenhaltungen: **GB-A- 1 542 444**

**Die Akte enthält technische Angaben, die nach dem Eingang der Anmeldung eingereicht wurden und die nicht in dieser Patentschrift enthalten sind.**

(73) Patentinhaber: **MOBAY CORPORATION, Mobay Road, Pittsburgh Pennsylvania 15205-9741 (US)**
Patentinhaber: **BAYER AG, D-5090 Leverkusen 1 Bayerwerk (DE)**

(72) Erfinder: **Jackman, Dennis E., 7350 Roe Circle, Prairie Village Kansas 66208 (US)**
Erfinder: **Westphal, Dietmar B., Dr., 8958 Woodstone, Lenexa Kansas 66219 (US)**
Erfinder: **Schmidt, Thomas, Dr., Ginsterweg 9, D-5657 Haan 1 (DE)**

(74) Vertreter: **Schumacher, Günter, Dr. et al, c/o Bayer AG Konzernverwaltung RP Patentabteilung, D-5090 Leverkusen 1 Bayerwerk (DE)**

**EP 0 168 766 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Umwandlung eines Thioethers in einen anderen Thioether, insbesondere ein Verfahren, in dem das Ausgangsmaterial ein S-substituierter Isothioharnstoff und das Endprodukt ein anderer S-substituierter Isothioharnstoff ist. Das neue Verfahren ermöglicht auch die Überführung eines Thioethers in das zugrundeliegende Thiol.

Die US-A 3 671 523 offenbart das ausserordentlich wirksame selektive Herbizid der Formel

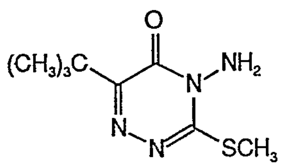

(A)

Die Verbindung wird durch Methylieren der entsprechenden Verbindung mit einem SH-Rest in der 3-Stellung mit Hilfe eines Methylierungsmittels wie Methylbromid oder Methyliodid hergestellt, wobei Methylchlorid viel niedrigere Ausbeuten liefert. Wenngleich das Verfahren gut arbeitet (wobei teilweise eine Methylierung mit Stickstoff-Atom in der 2-Stellung stattfindet), sind Methylbromid und -iodid doch relativ teuer.

Die US-A 4 457 774 offenbart ein ähnliches Verfahren zur Herstellung der Verbindung

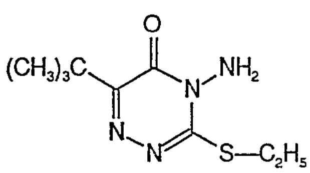

(B)

die ebenfalls selektiv herbizid wirkt, wobei Ethylbromid oder -iodid zur Verwendung gelangen, die noch teurer sind als Methylbromid bzw. -iodid und auch noch geringere Produktausbeuten liefern.

Der vorgenannte Stand der Technik umfasst ferner die Offenbarung mehrerer anderer Triazinone, die einen S-Alkyl-Substituenten in der 3-Stellung und andere Substituenten in der 4-Stellung und der 6-Stellung tragen; stellt man jedoch praktische wirtschaftliche Betrachtungen an, so werden diese Verbindungen unter Verwendung von teuren Alkylierungsmitteln hergestellt, die mit den entsprechenden 3-Thiolen zur Reaktion gebracht werden.

Dementsprechend ist es ein Ziel der vorliegenden Erfindung, andere S-substituierte Isothioharnstoffe aus S-Methyl-substituierten Isothioharnstoffen in einfacher, preisgünstiger Weise herzustellen.

Es ist ein weiteres Ziel der vorliegenden Erfindung, Thioether aus den entsprechenden Mercaptanen in preisgünstiger Weise herzustellen.

Noch ein weiteres Ziel der vorliegenden Erfindung ist es, S-substituierte Isothioharnstoffe in preisgünstiger Weise in die entsprechenden Thioharnstoffe umzuwandeln.

Diese und andere Ziele und Vorteile werden gemäss der vorliegenden Erfindung verwirklicht.

Gegenstand der vorliegenden Erfindung ist ein Verfahren zur Umwandlung eines die funktionelle Gruppe

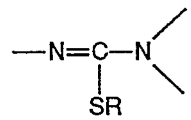

(C)

enthaltenden S-substituierten Isothioharnstoffs in den entsprechenden S-substituierten Isothioharnstoff mit der funktionellen Gruppe

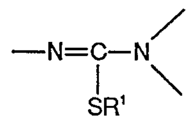

(D)

wobei die funktionellen Gruppen (C) und (D) jeweils Teil eines heterocyclischen Ringes sind und wobei in den Formeln (C) bzw. (D)

R ein Alkyl- oder Alkenyl-Rest mit jeweils bis zu 12 Kohlenstoffatomen oder ein Phenyl-, Naphthyl- oder Benzyl-Rest ist und

$R^1$ Wasserstoff, ein Alkyl- oder Alkenyl-Rest mit jeweils bis zu 12 Kohlenstoff-Atomen oder ein Phenyl-, Naphthyl- oder Benzyl-Rest ist, der jedoch von R verschieden ist, dadurch gekennzeichnet, dass man die Ausgangsverbindungen mit der funktionellen Gruppe (C), gegebenenfalls in Gegenwart eines Verdünnungsmittels und gegebenenfalls in Gegenwart eines Katalysators, mit einer Verbindung der Formel

$R^1$—SH (E)

umsetzt, wobei man gegebenenfalls die als Nebenprodukte gebildeten Verbindungen der Formel R–SH durch Destillation während oder nach der Reaktion aus dem Reaktionsgemisch entfernt.

Das Verfahren ist anwendbar speziell bei solchen Ausgangsstoffen, in denen die Struktur des S-substituierten Isothioharnstoffs Teil von substituierten Pyrimidinen (Uracilen), 1,3,5-Triazinen und dergleichen ist. Am meisten bevorzugt sind jedoch Ausgangsstoffe der Formel

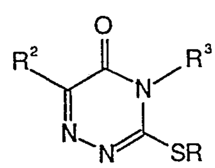

(F)

in der

R die oben angegebene Bedeutung hat,

$R^2$ ein Alkyl- oder Alkenyl-Rest mit bis zu 12 Kohlenstoff-Atomen oder ein Phenyl-Rest ist, der jeweils halogen-substituiert sein kann, und

$R^3$ ein $NH_2$-Rest oder ein Alkyl-, Alkenyl-, Aryl-, Aralkyl-, Alkylamino-, Dialkylamino-, Alkenylamino- oder Arylamino-Rest mit jeweils bis zu 12

Kohlenstoff-Atomen ist, und insbesondere diejenigen Ausgangsstoffe der Formel (F), in denen

R Methyl ist,

$R^2$ verzweigtes und gegebenenfalls halogensubstituiertes Alkyl mit bis zu 6 Kohlenstoff-Atomen ist (z.B. tert.-Butyl, Fluor- oder Chlor-tert.-butyl oder verzweigtes, ein quartäres Kohlenstoffatom enthaltendes Pentyl) und

$R^3$ $NH_2$ oder Alkyl, Alkylamino oder Dialkylamino mit jeweils bis zu 4 Kohlenstoff-Atomen ist, insbesondere $NH_2$ oder Methyl.

Bevorzugter Ausgangsstoff der Formel $R^1SH$ ist Ethylmercaptan.

Verwendet man als Ausgangsstoff der allgemeinen Formel (C) z.B. 4-Amino-6-tert.-butyl-3-methylthio-1,2,4-triazin-5(4H)-on, als Ausgangsverbindung der Formel (E) beispielsweise Ethylmercaptan und als Katalysator Kaliumhydroxid, so lässt sich der Reaktionsablauf nach dem erfindungsgemässen Verfahren (Alkylthio-Austauschreaktion) durch das folgende Formelschema wiedergeben:

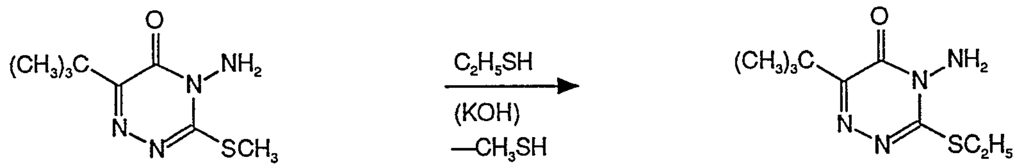


Vorteilhafterweise wird die Reaktion in einem Lösungsmittel und in Gegenwart eines Katalysators sowie unter Entfernung des Nebenprodukts RSH durch Destillation während oder nach der Reaktion durchgeführt, insbesondere dann, wenn RSH stärker flüchtig ist als $R^1SH$.

Die Reaktionstemperatur kann von Raumtemperatur oder noch tieferer Temperatur - z.B. 5°C - bis zur Siedetemperatur, je nach dem verwendeten Lösungsmittel, reichen, beträgt vorzugsweise jedoch 30°C bis 150°C.

Zu geeigneten Verdünnungsmitteln zählen inerte organische Lösungsmittel wie Toluol, halogenierte Kohlenwasserstoffe oder dergleichen, jedoch kann das Lösungsmittel auch einen Überschuss des Mercaptan-Reaktionspartners $R^1SH$ umfassen, sofern dieser flüssig ist. Der Einsatz von Wasser ist ebenfalls hilfreich, wenn das Reagenz oder das Endprodukt wasserlöslich ist.

Obwohl die Reaktionspartner in stöchiometrischen Mengen eingesetzt werden können, können auch grosse Überschüsse des einen der beiden Reaktionspartner eingesetzt werden, vorzugsweise des billigeren Reaktionsteilnehmers, um die Reaktion zur Vervollständigung zu treiben.

Mit Vorteil werden kleine Mengen saurer oder basischer Stoffe als Katalysatoren eingesetzt. Sie können in Mengen bis hinauf zur stöchiometrischen Menge vorliegen, jedoch sind im allgemeinen bereits so kleine Mengen wie 0,001 bis etwa 0,1 mol, vorzugsweise etwa 0,01 bis 0,05 mol, Katalysator auf 1 mol Isothioharnstoff geeignet. Basische Gruppen an dem reagierenden Molekül können selbst als innere Katalysatoren wirken.

Katalysatoren sind besonders nützlich, wenn niedrigsiedende Mercaptane mit 1 bis 4 Kohlenstoff-Atomen, z.B. Ethylmercaptan, eingesetzt werden. Zu bevorzugten Katalysatoren zählen Alkalimetallhydroxide oder Ammoniumhydroxid, Amine, basische Imide, basische Alkalimetall-Salze oder quartäre Ammoniumhydroxide sowie Säuren. Auch Ionenaustauschharz-Katalsyatoren sind einsetzbar, Fluoressigsäure und Kaliumcarbonat sind von geringem Vorteil, jedoch DABCO (Diazabicyclooctan), Triethylamin und Methylimidazol waren besser. Noch besser waren Tetramethylammoniumhydroxid, der Kronenether KF/18-Krone-6 und NaOH, jedoch die besten Ergebnisse wurden mit KOH erzielt.

Wie bereits erwähnt wurde, ist es auch möglich, wenn $R^1SH$ Schwefelwasserstoff ist und dieses im Überschuss in eine Lösung des Isothioharnstoffs eingeleitet wird, das Thiol oder Mercaptan zu bilden, d.h. $-SR$ in $-SH$ zu überführen.

Gemäss einem anderen Aspekt der vorliegenden Erfindung wird ein zweistufiges Verfahren verfügbar gemacht, das von dem Thioharnstoff

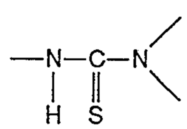

(G)

zu dem Isothioharnstoff

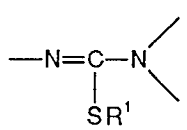

(D)

führt, wobei zuerst S-alkyliert wird - z.B. durch Umsetzung mit einem Alkylhalogenid, insbesondere Alkylbromid oder Alkyliodid -, um =S bzw. sein Isomer $-SH$ in $-SR$ umzuwandeln, und danach mit $R^1SH$ zu $-SR^1$ umgesetzt wird.

Die vorliegende Erfindung ist besonders wertvoll, wenngleich nicht auf einen solchen Einsatz beschränkt, für den Ersatz einer beliebigen Thiolether-Gruppe zur Herstellung der nachstehenden Verbindungen neben denjenigen, die im Folgenden beispielhaft genannt werden:

1-Amino-6-ethylthio-3-(2,2-dimethylpropyl)-1,3,5-triazin-2,4(1H,3H)-dion,
4-Amino-6-(chloro-tert.-butyl)-3-propargylthio-1,2,4-triazin-5-on,
4-Amino-6-(fluoro-tert.-butyl)-3-allylthio-1,2,4-triazin-5-on,
4-Amino-6-(fluoro-tert.-butyl)-3-methylthio-1,2,4-triazin-5-on,

4-Amino-6-(1,1-bis-fluoromethyl-ethyl)-3-(buten-2'-yl-thio)-1,2,4-triazin-5-on,
4-Amino-6-(ethoxy-tert.-butyl)-3-propargylthio-1,2,4-triazin-5-on,
6-(2,3-Dimethyl-but-2-yl)-4-methyl-3-methylthio-1,2,4-triazin-5-on,
4-Amino-6-cyclobutyl-3-methylthio-1,2,4-triazin-5-on,
6-Cyclobutyl-4-isopropylidenimino-3-methylthio-1,2,4-triazin-5-on.

Die nachfolgenden Beispiele dienen zur weiteren Erläuterung der Erfindung.

Beispiel 1

Eine Mischung aus 230 g (1 mol bei 93% Wirkstoff) 4-Amino-6-tert.-butyl-3-methylthio-1,2,4-triazin-5-on und 310 g (5 mol) Ethylmercaptan und 0,5 g zerkleinertes festes KOH wurde unter Rühren zum Rückfluss an einem bei 10°C betriebenen Kühler erhitzt. Die durch den Kühler hindurchgehenden Dämpfe (Methylmercaptan und Ethylmercaptan) wurden in einer Trockeneis-Falle oder einer Ätzalkali-Falle gesammelt. Die Reaktion wurde so lange fortgeführt, bis dünnschichtchromatographisch festgestellt wurde, dass das Ausgangsmaterial verschwunden war (gewöhnlich 2 bis 8 h). Das unumgesetzte Ethylmercaptan wurde dann abdestilliert, während etwa 600 ml warmes (40°C) Wasser mit solcher Geschwindigkeit zugesetzt wurden, dass die Wasser-Zugabe vollständig war, wenn etwa 2/3 des Mercaptans abdestillliert waren. Die Destillation wurde fortgesetzt, bis etwa 100 ml Wasser bei 100°C destilliert waren. Die Lösung wurde dann unter raschem Rühren abkühlen gelassen. Das Produkt kristallisierte bei 60°C bis 70°C aus und wurde bei 20°C filtriert, mit Wasser gewaschen und dann getrocknet. Das Endprodukt, 4-Amino-6-tert.-butyl-3-ethylthio-1,2,4-triazin-5-on, wurde in nahezu quantitativer Ausbeute erhalten und enthielt weniger als 1% der 3-Methylthio-Ausgangsverbindung.

Beispiel 2

Eine Lösung von 2,14 g (0,01 mol) 4-Amino-6-tert.-butyl-3-methylthio-1,2,4-triazin-5-on und 1 Tropfen Triethylamin in 25 ml Toluol wurde 0,5 h mit überschüssigem $H_2S$-Gas behandelt. Die entsprechende 3-Thio-Verbindung wurde in quantitativer Ausbeute gebildet.

Beispiel 3

10 g (0,0515 mol) 4-Amino-6-tert.-butyl-3-methylthio-1,2,4-triazin-5-on, 35 g (0,257 mol) m-Thiocresol und 0,2 g Kaliumhydroxid wurden 8 h in absolutem Ethanol zum Rückfluss erhitzt. Das Produkt, 4-Amino-6-tert.-butyl-3-(3'-methylphenylthio)-1,2,4-triazin-5-on, wurde durch Kristallisation aus Ethanol-Wasser erhalten (Schmelzpunkt 150–153°C).

Beispiel 4

Eine Mischung aus 10,7 g (0,05 mol) 4-Amino-6-tert.-butyl-3-methylthio-1,2,4-triazin-5-on und 35 g (0,17 mol) Dodecanthiol wurde in einem leichten Stickstoff-Strom 8 h auf 120°C erhitzt. Cyclohexan wurde zugesetzt, und das Produkt (4-Amino-6-tert.-butyl-3-dodecylthio-1,2,4-triazin-5-on) kristallisierte in nahezu quantitativer Ausbeute; Schmelzpunkt 64–66°C.

Beispiel 5

4-Amino-6-methyl-3-methylthio-1,2,4-triazin-5-on (8,6 g; 0,05 mol), tert.-Butylmercaptan (27 g; 0,3 mol) und 1,5 g 20-proz. Tetramethylammoniumhydroxid wurden 60 h unter Rückfluss erhitzt. Die Lösung wurde zur Trockne eingedampft, 35 ml Hexan von 60°C wurden hinzugefügt, wodurch eine Lösung entstand; diese wurde auf Raumtemperatur gekühlt, wodurch eine Menge von 7,5 g (70%) der entsprechenden 3-tert.-Butylthio-Verbindung vom Schmelzpunkt 124–125°C erhalten wurde. Weiteres Produkt verblieb in der Mutterlauge.

Beispiel 6

2-Methylthio-3-amino-6-methyluracil (1,1 g; 0,0064 mol), Dodecanthiol (10 g; 0,0495 mol) und einige Tropfen Tetramethylammoniumhydroxid-Lösung (25%) wurden 3 h auf 140°C erhitzt. Verdünnung mit Heptan (30 ml) und Abkühlen lieferte 1,7 g (80% Ausbeute) des Produkts, 2-Dodecylthio-3-amino-6-methyluracil; Schmelzpunkt 80°C. Die Reaktion wird folgendermassen veranschaulicht:

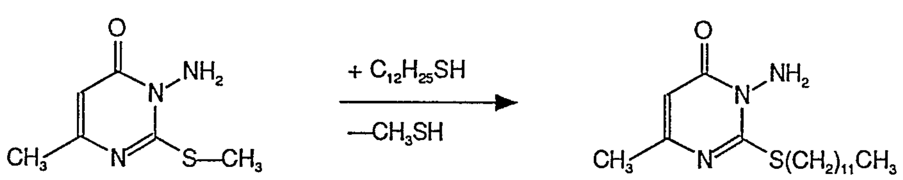


Beispiel 7

1-Amino-3-(2,2-dimethylpropyl)-6-ethylthio-1,3,5-triazin-2,4(1H,3H)dion (5 g; 0,019 mol), Cyclohexanthiol (6,0 g; 0,05 mol) und 2 Tropfen Tetramethylammoniumhydroxid-Lösung (25%) wurden 4 h auf 150°C erhitzt. Heptan (20 ml) wurde langsam zugesetzt, und 5,7 g (94%) des Produkts, 1-Amino-3-(2,2-dimethylpropyl)-6-cyclohexylthio-1,3,5-triazin-2,4-(1H,3H)dion, wurden durch Filtration erhalten. Die Reaktion wird folgendermassen veranschaulicht:

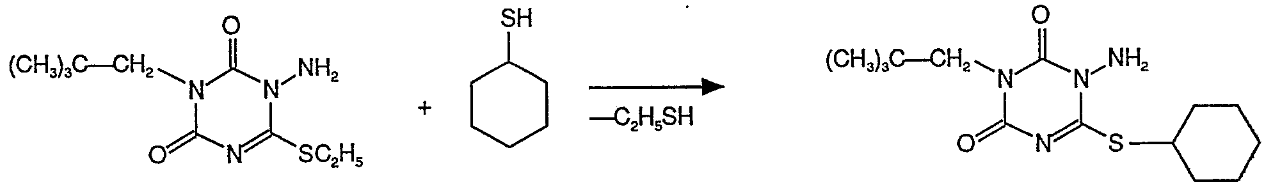


**Patentansprüche**

1. Verfahren zur Umwandlung eines die funktionelle Gruppe

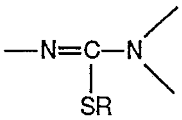 (C)

enthaltenden S-substituierten Isothioharnstoffs in den entsprechenden S-substituierten Isothioharnstoff mit der funktionellen Gruppe

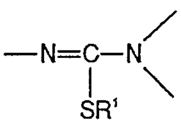 (D)

wobei die funktionellen Gruppen (C) und (D) jeweils Teil eines heterocyclischen Ringes sind und wobei in den Formeln (C) bzw. (D)

R ein Alkyl- oder Alkenyl-Rest mit jeweils bis zu 12 Kohlenstoffatomen oder ein Phenyl-, Naphthyl- oder Benzyl-Rest ist und

$R^1$ Wasserstoff, ein Alkyl- oder Alkenyl-Rest mit jeweils bis zu 12 Kohlenstoff-Atomen oder ein Phenyl-, Naphthyl- oder Benzyl-Rest ist, der jedoch von R verschieden ist, dadurch gekennzeichnet, dass man die Ausgangsverbindungen mit der Funktionellen Gruppe (C), gegebenenfalls in Gegenwart eines Verdünnungsmittels und gegebenenfalls in Gegenwart eines Katalysators, mit einer Verbindung der Formel

$R^1$—SH (E)

umsetzt, wobei man gegebenenfalls die als Nebenprodukte gebildeten Verbindungen der Formel R-SH durch Destillation während oder nach der Reaktion aus dem Reaktionsgemisch entfernt.

2. Verfahren nach Anspruch 1, dadurch gekennzeichnet, dass eine Ausgangsverbindung der Formel

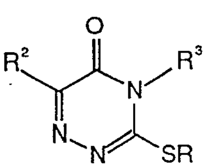 (F)

in der

R die in Anspruch 1 angegebene Bedeutung hat,

$R^2$ ein Alkyl- oder Alkenyl-Rest mit jeweils bis zu 12 Kohlenstoffatomen oder ein Phenyl-Rest ist, der jeweils halogen-substituiert sein kann, und

$R^3$ ein $NH_2$-Rest oder Alkyl-, Alkenyl-, Aryl-, Aralkyl-, Alkylamino-, Dialkylamino-, Alkenylamino oder Arylamino-Rest mit jeweils bis zu 12 Kohlenstoffatomen ist, eingesetzt wird.

3. Verfahren nach Anspruch 2, dadurch gekennzeichnet, dass in Formel (F)

R Methyl ist,

$R^2$ verzweigtes und gegebenenfalls halogensubstituiertes Alkyl mit bis zu 6 Kohlenstoffatomen ist und

$R^3$ $NH_2$ oder ein Alkyl-, Alkylamino- oder Dialkylamino-Rest mit jeweils bis zu 4 Kohlenstoffatomen ist.

4. Verfahren nach Anspruch 1, dadurch gekennzeichnet, dass man bei Temperaturen zwischen etwa 5°C und dem Siedepunkt des verwendeten Verdünnungsmittels arbeitet.

5. Verfahren nach Anspruch 4, dadurch gekennzeichnet, dass man bei Temperaturen zwischen etwa 30°C und 150°C arbeitet.

6. Verfahren nach Anspruch 1, dadurch gekennzeichnet, dass man die Umsetzung in Gegenwart eines Katalysators durchführt und dass man als Katalysator ein Alkalihydroxid oder Ammoniumhydroxid, ein Amin, ein basisches Imid, ein basisches Alkalimetall-Salz oder ein quartäres Ammoniumhydroxid oder eine Säure verwendet.

7. Verfahren nach Anspruch 6, dadurch gekennzeichnet, dass man als Katalysator Kaliumhydroxid einsetzt.

8. Verfahren nach Anspruch 1, dadurch gekennzeichnet, dass R-SH stärker flüchtig ist als $R^1$-SH und dass R-SH während oder nach der Reaktion aus dem Reaktionsgemisch abdestilliert wird.

9. Verfahren nach Anspruch 3, dadurch gekennzeichnet, dass in Formel (F)

R für $CH_3$ steht,
$R^2$ für $(CH_3)_3C$ steht und
$R^3$ für $NH_2$ steht,

dass die Reaktion in überschüssigem Ethylmercaptan als Reaktionspartner $R^1$-SH (E) und als Verdünnungsmittel und in Gegenwart von Kaliumhydroxid als Katalysator durchgeführt wird und dass das Nebenprodukt $CH_3SH$ während oder nach der Reaktion aus dem Reaktionsgemisch abdestilliert wird.

## Claims

1. Process for converting an S-substituted isothio urea containing the functional group

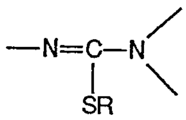

(C)

into the corresponding S-substituted isothio urea having the functional group

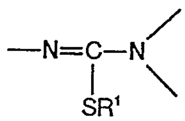

(D)

where the functional groups (C) and (D) in each case are part of a heterocyclic ring and where in the formulae (C) and (D)

R is an alkyl or alkenyl radical having in each case up to 12 carbon atoms or is a phenyl, naphthyl or benzyl radical and

$R^1$ is hydrogen, an alkyl or alkenyl radical having in each case up to 12 carbon atoms or is a phenyl, naphthyl or benzyl radical which is, however, different from R, characterized in that the starting compounds having the functional group (C) are reacted with a compound of the formula

$R^1$-SH (E)

if appropriate in the presence of a diluent and if appropriate in the presence of a catalyst, in which process the compounds of the formula R-SH, which are formed as by-products, are optionally removed from the reaction mixture by distillation during or after the reaction.

2. Process according to Claim 1, characterized in that a starting compound of the formula

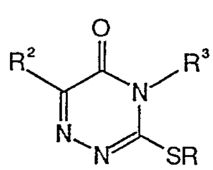

(F)

in which

R has the meaning given in Claim 1,

$R^2$ is an alkyl or alkenyl radical having in each case up to 12 carbon atoms or is a phenyl radical, each of which radicals can be halogen-substituted, and

$R^3$ is an $NH_2$ radical or is an alkyl, alkenyl, aryl, aralkyl, alkylamino, dialkylamino, alkenylamino or arylamino radical having in each case up to 12 carbon atoms, is employed.

3. Process according to Claim 2, characterized in that, in formula (F),

R is methyl,

$R^2$ is branched and optionally halogen-substituted alkyl having up to 6 carbon atoms and

$R^3$ is $NH_2$ or is an alkyl, alkylamino or dialkylamino radical having in each case up to 4 carbon atoms.

4. Process according to Claim 1, characterized in that the process is carried out at temperatures between approximately 5 °C and the boiling point of the diluent used.

5. Process according to Claim 4, characterized in that the process is carried out at temperatures between approximately 30 °C and 150°C.

6. Process according to Claim 1, characterized in that the reaction is carried out in the presence of a catalyst and in that an alkali metal hydroxide or ammonium hydroxide, an amine, a basic imide, a basic alkali metal salt or a quarternary ammonium hydroxide or an acid is used as catalyst.

7. Process according to Claim 6, characterized in that potassium hydroxide is employed as catalyst.

8. Process according to Claim 1, characterized in that R-SH is more volatile than $R^1$-SH and in that R-SH is distilled off from the reaction mixture during or after the reaction.

9. Process according to Claim 3, characterized in that, in formula (F),

R represents $CH_3$,

$R^2$ represents $(CH_3)_3C$

$R^3$ represents $NH_2$,

in that the reaction is carried out in excess ethylmercaptane as the reactant $R^1$-SH (E) and as the diluent and in the presence of potassium hydroxide as the catalyst, and in that the by-product $CH_3SH$ is distilled off from the reaction mixture during or after the reaction.

## Revendications

1. Procédé en vue de la transformation d'une isothiourée substituée sur l'atome de soufre contenant le groupe fonctionnel

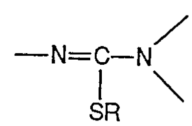

(C)

en isothiourée correspondante substituée sur l'atome de soufre, et comportant le groupe fonctionnel

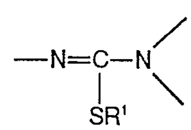

(D)

procédé dans lequel les groupes fonctionnels (C) et (D) font chacun partie d'un noyau hetérocyclique, tandis que dans les formules (C) ou (D)

R représente un radical alkyle, ou un radical alcenyle, contenant chacun jusqu'à 12 atomes de carbone, ou un radical phényle, un radical naphtyle ou un radical benzyle, et

$R^1$ représente un atome d'hydrogène, un radical alkyle ou un radical alcenyle contenant chacun jusqu'à 12 atomes de carbone, ou un radical phényle, un radical naphtyle ou un radical benzyle, qui est toutefois différent de R, caractérisé en ce que l'on fait réagir les composés de départ contenant le groupe fonctionnel (C), éventuellement en présence d'un diluant, et éventuellement, en présence d'un catalyseur, avec un composé de formule

$R^1$—SH (E)

tandis que, éventuellement, par distillation, pendant ou après la réaction, hors du mélange reactionnel, on élimine les composés de formule R-SH, que l'on a obtenu sous forme de sous-produit.

2. Procédé selon la revendication 1, caractérisé en ce que l'on utilise un composé de départ de formule

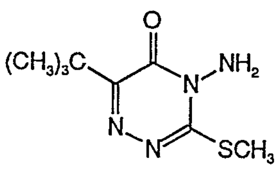

(F)

dans laquelle

R à la signification indiquée dans la revendication 1.

$R^2$ représente un radical alkyle ou un radical alcényle contenant chacun jusqu'à 12 atomes de carbone, ou un radical phényle pouvant chacun être substitué par un atome d'halogène, et

$R^3$ représente un radical $NH_2$ ou un radical alkyle, un radical alcényle, un radical aryle, un radical aralkyle, un radical alkylamino, un radical dialkylamino, un radical alcénylamino, ou un radical arylamino contenant chacun jusqu'à 12 atomes de carbone.

3. Procédé selon la revendication 2, caractérisé en ce que, dans la formule (F)

R représente un groupe méthyle,

$R^2$ représente un groupe alkyle à chaîne ramifiée et éventuellement substitué par un atome d'halogène, contenant jusqu'à 6 atomes de carbone, et

$R^3$ représente $NH_2$ ou un radical alkyle un radical alkylamino, ou un radical dialkylamino, contenant chacun jusqu'à 4 atomes de carbone.

4. Procédé selon la revendication 1, caractérisé en ce que l'on travaille à des températures comprises dans l'intervalle allant d'environ 5 °C à la température d'ébullition du diluant utilisé.

5. Procédé selon la revendication 4, caractérisé en ce que l'on travaille à des températures comprises dans l'intervalle allant d'environ 30 °C à 150 °C.

6. Procédé selon la revendication 1, caractérisé en ce que l'on effectue la réaction en présence d'un catalyseur et en ce que l'on utilise, comme catalyseur, un hydroxyde de métal alcalin ou l'hydroxyde d'ammonium, une amine, un imide basique, un sel basique de métal alcalin ou un hydroxyde d'ammonium quaternaire, ou encore un acide.

7. Procédé selon la revendication 6, caractérisé en ce qu'on utilise l'hydroxyde de potassium comme catalyseur.

8. Procédé selon la revendication 1, caractérisé en ce que le groupe R-SH est plus volatil que le groupe $R^1$-SH et en ce que, par distillation, on sépare le groupe R-SH, pendant ou après la réaction, hors du mélange réactionnel.

9. Procédé selon la revendication 3, caractérisé en ce que, dans la formule (F)

R représente le groupe $CH_3$,

$R^2$ représente le groupe $(CH_3)_3C$ et

$R^3$ représente le groupe $NH_2$,

en ce qu'on effectue la réaction en présence d'un excès d'éthyl-mercaptan, comme partenaire reactionnel $R^1$-SH (E) et comme diluant, ainsi qu'en présence d'hydroxyde de potassium, comme catalyseur, et en ce que, par distillation, hors du mélange reactionnel, on sépare le sous-produit, à savoir $CH_3SH$, pendant ou après la réaction.